# EUROPEAN PATENT SPECIFICATION

(11) **EP 1 177 779 B1**
(45) Date of publication and mention of the grant of the patent: **20.05.2009**
(21) Application number: 01118408.2
(22) Date of filing: 31.07.2001
(51) Int. Cl.: A61F 2/06

(54) **Stent and method of manufacturing**
Stent und Verfahren zu seiner Herstellung
Stent et procédé de fabrication

(30) Priority: 31.07.2000 JP 2000231267
(43) Date of publication of application: 06.02.2002
(73) Proprietor: Mani, Inc., Shioya-gun, Tochigi-ken (JP)
(72) Inventor: Matsutani, Masaaki, Shioya-gun, Tochigi-ken (JP); Fukuda, Masatoshi, Shioya-gun, Tochigi-ken (JP); Fukuda, Shoichi, Shioya-gun, Tochigi-ken (JP)
(74) Representative: Patentanwälte Westphal, Mussgnug & Partner

(56) References cited:
- US-A- 5 147 370
- US-A- 5 554 181
- US-A- 5 766 237
- US-A- 5 830 179

## Description

### BACKGROUND OF THE INVENTION

### 1. Field of the Invention

This invention relates to a method for manufacturing a stent for treating internal tubular organs such as a blood vessel, and more particularly, for treating aneurysm.

### 2. Description of Related Art

Inside a body, there are many tubular tissues, such as a blood vessel, a bile duct, a ureter, an esophagus, or the like; such organs bear a possibility of characteristic ailments such as stenosis or occlusion. For example, in respect of blood vessels, there are possibilities of ailments such as stenosis, occlusion, aneurysm, varicosity, and the like. More particularly, since aneurysm is a serious ailment where a rupture of a blood vessel causes excessive bleeding, prompt treatment is to be required, and various tools have been developed for treating thus ailment effectively.

Lately, the use of a metallic cylindrical tool called a stent is increasingly used for treating a portion of blood vessel stenosis or for treating aneurysm. For example, when treating aneurysm, a stentgraf, which covers the stent with an artificial blood vessel, is used; such stentgraf is positioned in place at the inner side of the aneurysm so that pressure of blood would not affect the aneurysm.

As for examples regarding the kinds of stents, there is a cylindrical stent made from a metal material (representatively from stainless steel) or a stent having a metal wire bent in a zigzag manner while at the same time having a cylindrical shape. US-A-5 766 237, US-A-5 554 181 and US-A-5 147 370 all disclose such stents. Such stents are delivered to an affected portion internally through a blood vessel and positioned in place when reaching the affected portion.

In a case of stationing the stent at the affected portion, the stent is diametrally shrunk and confined within a long tubular delivery kit, in which the delivery kit has a guiding wire inserted therethrough; and then, starting from the guiding wire, the delivery kit is guided through a blood vessel from a root of a leg till reaching the affected portion, and then, when reaching the affected portion, the delivery kit is retracted to release the stent from confinement. After being released from confinement, the stent diametrally self-expands, and is positioned in place at the affected portion in thus expanded state for protecting the blood vessel.

The stent being positioned in place should preferably be stuck closely to an inner wall of a tissue without having any gap and should apply a suitable expanding force upon the inner wall. For example, having a gap between the stent and the inner wall of the tissue would be the cause for disorder of blood flow and result to a thrombus and the like; further, there would be a risk of the stent becoming out of place from the affected portion if the expanding force is too weak, and there would be a risk of the tissue becoming constricted and result to necrosis if the expanding force is too strong.

In general, the thickness and shape of a tubular living-body tissue for each individual are different. However, there is a problem that the stent cannot always correspond to the different thickness and shape of an organic tubular tissue for each individual since the stent has a predetermined diameter and straight line shape. Accordingly, heightening the flexibility of a straight shaped stent to allow the stent to become closely stuck to the inner wall of the tissue, is proposed.

Nevertheless, in correspondence with the heightening the flexibility of the stent, the strength of the stent would decrease and result to a decrease in expanding force; further, the stent would become easily compressed in an axial direction to cause a risk of becoming drawn toward an aneurysm.

It is an object of this invention to provide a stent manufactured based on the information regarding tubular living-body tissue for each individual, and to provide a method of manufacturing the stent.

### SUMMARY OF THE INVENTION

In means to solve the aforementioned problems, the stent for this invention is formed by a mechanical method to match with an inner shape of a tubular living-body tissue of each individual, in accordance with shape-information regarding the tubular living-body tissue of each individual, wherein the shape-information is obtained by a non surgical method.

Since the shape of the foregoing stent matches with the shape of the tubular living-body tissue of the targeted individual, the stent could stick closely to an inner wall of the tubular tissue while applying thereto a suitable expanding force and could be steadily positioned in place, when the stent is positioned in place at an internal targeted portion. More particularly, the tubular tissue slightly bends and bears changes in diameter depending on the individual. Accordingly, the stent, being formed in accordance with the shape-information of each individual, could be stuck closely to the inner wall of the tubular tissue in a satisfactory manner when precisely positioned in place at the targeted portion.

As a means for obtaining tubular living-body information of an individual, there are methods such as a method using roentogen, ultrasonic tomogram (US), or computer tomography (CT), or magnetic resonance imaging (MRI), in which three dimensional information could be extracted from the information from such methods. Accordingly, based on such information, the aimed stent could be manufactured by a mechanical method using for example a numerical control (NC) machine tool, such as an NC lathe, an NC milling machine, or a machining center.

Another stent regarding this invention is formed to match with an inner shape of a tubular living-body tissue of each individual by performing shape-memorization in correspondence to a master, in which the master has an inner surface or outer surface with a shape being substantially similar to the inner shape of the tubular living-body tissue of each individual and being formed in accordance with shape-information obtained by a mechanical method.

In respect of the foregoing stent, since shape-memorization is performed in correspondence to the master being formed in accordance with the mechanically obtained shape-information of the tubular tissue of an individual, the stent having returned back to the memorized shape bears a shape substantially equal to that of the targeted tubular tissue of the individual, and maintains a steady state in a manner applying a suitable expanding force upon the tubular tissue when positioned in place at a targeted portion.

When performing treatment by positioning the stent in place at the portion targeted for treatment, in means to heighten the sticking degree upon the targeted portion, the outer diameter of the stent is required to be substantially equal to or slightly larger (although there is a difference depending on the shape of the targeted portion or the like, up till approximately two times larger than the shortest diameter of the targeted portion) than the inner diameter of the portion targeted for treatment. In such case, since the outer diameter of the stent would be the sum of the outer surface or inner surface of the master added to the thickness of the material comprising the stent, the diameter for the outer surface or the inner surface of the master is determined by taking into consideration the thickness of the stent. Accordingly, the inner surface or outer surface of the master is required to be substantially similar to the shape of the inner surface of the tubular tissue (hereinafter the same).

A method of manufacturing a stent regarding this invention serves to manufacture a stent matching with the inner shape of the tubular living-body tissue of each individual by comprising the steps of: obtaining inner shape-information regarding tubular living-body tissue of each individual by using a non surgical method; making a master in accordance with the shape-information in which the master has a three dimensional inner surface or outer surface substantially similar to the inner shape of the tubular living-body tissue; and performing shape-memorization in a manner corresponding to the master.

With the foregoing method of manufacturing a stent, a three dimensional master could be made in according with the shape-information regarding the tubular living-body tissue of each individual, wherein the shape-information is obtained by a non surgical method. By performing shape-memorization using the master, a stent, which is capable of returning back to the memorized shape substantially equal to that of the tubular tissue of the targeted individual, could be manufactured.

### BRIEF DESCRIPTION OF THE DRAWINGS

The above and other objects and features of the invention are apparent to those skilled in the art from the following preferred embodiments thereof when considered in conjunction with the accompanied drawings, in which:
FIG.1 is an explanatory view showing an example of a stent for use during treatment of aneurysm;
FIG.2 is a view showing a thoracic descending aorta as an example of a portion for treatment;
FIG.3 is a view for explaining a shape of a master;
FIG.4 is an explanatory front view showing a state where a wire made from a shape memorizing alloy is wrapped around a master;
FIG.5 is view V of FIG.4; and
FIG.6 is view VIof FIG.4.

### DESCRIPTION OF THE PREFERRED EMBODIMENTS

The stent of this invention is processed for matching with an inner shape of a tubular tissue by obtaining three dimensional shape-information of tubular living-body tissue, that is, an affected portion, by means of a mechanical method such as roentogen, ultrasonic tomography (US), computer tomography (CT), magnetic resonance imaging (MRI), and then, using a mechanical method such as a machine tool controlled on the basis of the information.

Accordingly, the processed stent has no interchangeability between other patients, and is formed as an exclusive stent only for a particular patient. Therefore, when positioned in place at the affected portion, the stent practically matches with the inner shape of the tubular tissue of such portion to allow the stent to contact and apply a suitable expanding force upon an inner surface of the tubular tissue, and to allow the stent to maintain a steady position, further, there is no risk of causing trouble such as necrosis upon the tubular tissue. Further, there is no risk of affecting the flow of bodily fluid since the inner surface of the stent practically matches with the inner surface of the affected portion.

The stent is not inserted and positioned in place by means of cutting open the targeted portion for treatment, but instead, is guided inside from the external, conveyed and positioned in place when reaching the targeted portion for treatment. Accordingly, during the process of conveying the stent to the targeted portion for treatment, it is preferable to allow the stent to maintain a shrunken state having a small diameter, and to allow expansion to the original diameter of the stent when reaching the targeted portion for treatment.

As for the material of the stent having the aforementioned characteristics, a metal material bearing elasticity and flexibility without harming living body tissue such as shape-memorizing alloy of Nickel (Ni) and Titanium (Ti), or a synthetic resin material capable of releasing expanding force by having a suitable elasticity without harming living body tissue, could be selectively used.

For example, the stent could be manufactured by being processed three dimensionally with use of a machine tool such as a numerically controlled (NC) milling machine or a machining center. Further, the aimed stent could be manufactured by bending with use of a pipe processing machine such as an NC bending machine or an NC spinning machine, and drawing processing according to circumstance.

When a shape-memorizing alloyed block or pipe is selected as the material for the stent, the stent could be manufactured by using the foregoing methods. Further, when a shape-memorizing alloy wire is selected as the material for the stent, the stent could be manufactured by forming a three dimensional master based on shape-information of the tubular tissue, and then performing shape-memorization by wrapping the wire along the master and maintaining thus wrapped state. Further, when a net-like shape-memorized alloy is used as the material, the aimed stent could be manufactured by using the foregoing method.

With the foregoing stent, a stentgraf could be formed to allow the stentgraf to be positioned in place at the affected portion for performing the aimed treatment, by covering the shape-memorizing alloy wire, which has been shape-memorized by being wrapped along the master, with an artificial blood vessel or the like, in correspondence to the subject tubular tissue.

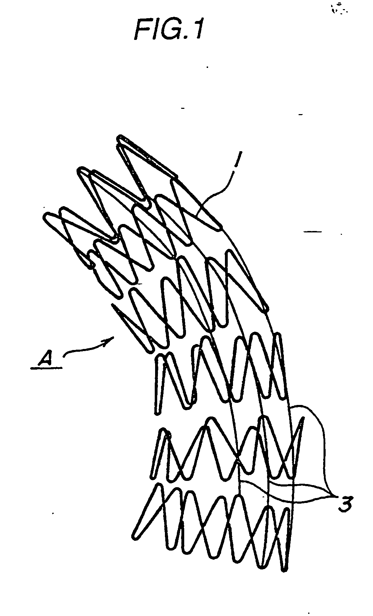

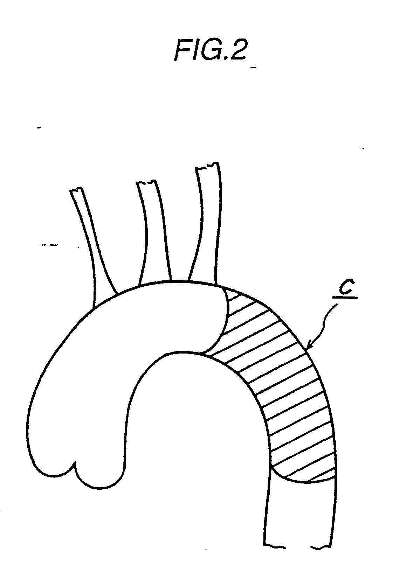

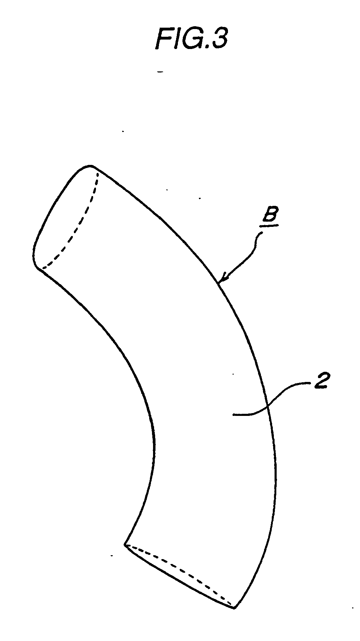

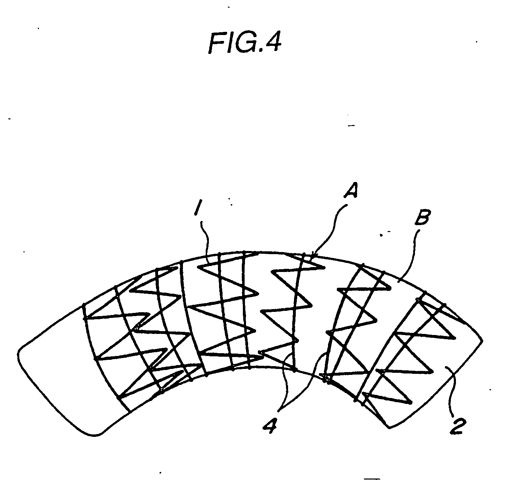

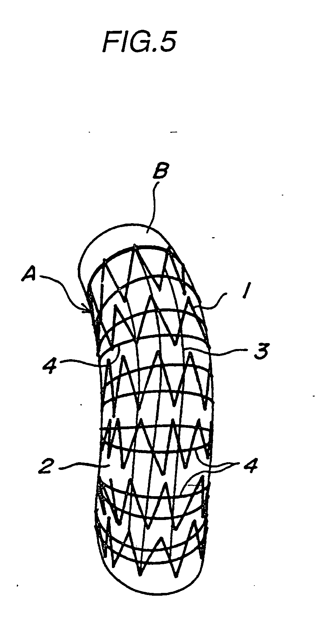

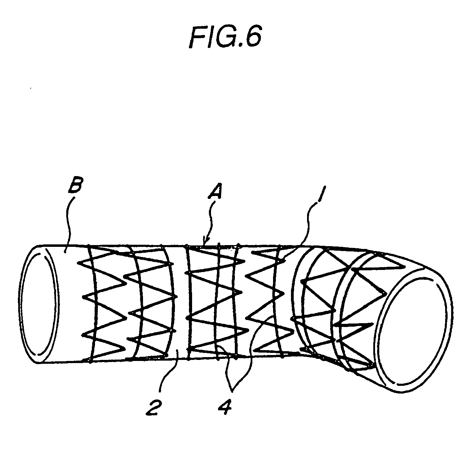

A preferable embodiment of the stent along with a method of manufacturing such stent will hereinafter be described with reference to the drawings. FIG.1 is an explanatory view showing an example of a stent for use during treatment of aneurysm; FIG.2 is a view showing a thoracic descending aorta as an example of a portion for treatment; FIG.3 is a view for explaining a shape of a master; FIG.4 is an explanatory front view showing a state where a wire made from a shape memorizing alloy is wrapped around a master; FIG.5 is view V of FIG.4; and FIG.6 is view VIof FIG.4.

It is to be noted that this embodiment of a stent A is memorized with a zigzag shape in which each tip portion of the zigzag are bent with a prescribed bending radius, as shown in FIG.1. Although, the tip portions are shown in an acutely intersecting manner in FIG.4 through FIG.5, the tip portions are also bent as in FIG.1.

The stent is applied to a tubular tissue targeted for treatment or a portion of such tubular tissue. Since the size and the measurement for the stent differs depending on the tubular tissue targeted for treatment or the portion thereof, the stent does not essentially bear interchangeability, but instead should be processed individually in accordance with the tubular tissue targeted for treatment or the portion thereof. However, since it would become complicated to describe the shape of the stent in correspondence with each targeted tubular tissue or the portion thereof, a stent A for treating aneurysm at a thoracic descending aorta (hereinafter referred as "affected portion C") as shown in FIG.2 will be described in this embodiment.

In this embodiment, the stent A has a wire 1 being wrapped around a targeted affected portion C in a zigzag manner and is formed substantially equal to a three dimensional shape of the affected portion C and formed substantially equal to the measurement of an inner surface of the affected portion. A stentgraf is formed by covering the stent A with an artificial blood vessel in which the stentgraf is positioned in place at the affected portion C for enabling treatment by removal of the strain applied upon the aneurysm.

The thickness of the wire 1 comprising the stent A is not to be limited, and should be selected depending on the ability to withstand the force applied upon the affected portion targeted for placement. The material of the stent A is a shape-memorizing alloy which preferably.

The stent A is formed based on shape-information of tubular tissue regarding the affected portion C for each individual requiring treatment. The shape-information for each subject individual is obtained selectively by means such as roentogen rays, ultra sonic tomogram (US), computer tomographic image (CT), or a magnetic resonance image (MRI).

The foregoing images could be obtained by respectively applying and operating devices upon the subject individual, and in correspondence to the taken images, three dimensional shape-information regarding the affected portion C (including information of shape and measurement) could be obtained by processing the data outputted from the devices.

Accordingly, the stent A being formed based on the obtained three dimensional shape-information of affected portion C, is inserted inside a delivery kit in a diametrally shrunken state, and then guided inside from outside of the body, and then taken out from the delivery kit when reaching the affected portion C so as to diametrally expand back to the original diameter and stick closely to the inner surface of the affected portion C while applying a suitable expanding force thereto. In this state, no gap is formed between the stent A and the inner surface of the tubular tissue, and excessive pressure would not be applied upon the tissue.

Therefore, the stent A could maintain being steadily positioned in place at the affected portion, and aneurysm could be protected from the pressure of blood at the affected portion C.

The stent regarding this invention is not to be limited to the stent A formed with the wire 1 in a wrapping manner, but is also to include a net-like stent, or a coiling stent. In a case of using such net-like stent or coiling stent, such stents could be formed by using a machine tool controlled according to the three dimensional shape-information of affected portion C; or could be formed with a shape-memorizing alloy such as a Ni-Ti alloy by performing shape-memorization in a manner where the stent is constrained according to the three dimensional shape-information of affected portion C.

Next, a process of forming the stent A by using a wire 1 made from a shape-memorizing alloy of Ni-Ti will be explained. In this embodiment, the wire 1 for the stent A is comprised of nickel (Ni) and titanium (Ti) in which nickel is of 56.06 % weight and the remaining thereof is titanium; further, the wire 1 has a diameter of 0.4mm.

A master B having a form matching with an aimed shape is used, when bending and forming the wire 1 made from the shape-memorizing alloy of Ni-Ti into a cylindrical shape. An outer surface 2 of the master B has a three dimensional shape which is substantially similar to a shape of an inner surface of the affected portion C targeted for the stent A.

The outer surface of the master B is formed by using a mechanical method in accordance with the three dimensional shape-information of the affected portion B being the individual's targeted portion for treatment, wherein the shape-information is obtained by mechanical methods such as roentogen rays, US, CT, MRI. Accordingly, the inner surface of the affected portion C could be altered to the outer surface 2 of the master B having a substantially similar shape to the inner surface so as to form the stent A with a shape in correspondence to that of outer surface 2; subsequently, the inner surface of the affected portion C and the inner surface of the stent A could precisely match when the stent A is positioned in place at the affected portion C.

After the thus formed outer surface 2 of the master B is wrapped in a zigzag manner as well as a belt-like manner by the wire 1, as shown in FIG.4 through FIG.6, a shape-memorization process is performed. The measurement regarding the detail of the zigzag shaped wire 1 is not to be limited in particular, but is to be set in accordance with conditions such as the thickness of the affected portion C or the bending shape thereof. For example, when the affected portion C is of a substantially thick portion, as in this embodiment, the length of the zigzag shape in the axial direction is set to approximately 25mm.

Further, the zigzagging peak portions are formed by bending the wire 1. The manner in bending the peak portions have a large influence on performance during the diametral shrinkage of the stent A. That is, the smaller the bending radius of the peak portion is, the higher the diametral shrinking performance would become and the easier the fracture of the wire would become. Further, the larger the bending radius of the peak portion is, the poorer the diametral shrinking performance would become and the more difficult the fracture of the wire would become. Accordingly, since the diametral shrinkage and the tendency of fracture are contradictory characteristics, and the most suitable bending radius is set depending on conditions such as the function or portion of the tubular tissue subject for treatment. In this embodiment, the bending radius of the peak portion is set to 0.5mm.

One method of wrapping the outer surface 2 of the master B with the wire 1 is performed by bending a single wire 1 in a zigzag shape and wrapping in spiral manner, while the other method is performed by forming a wire 1, which is already shape-memorized with a zigzag shape, into a ring-shape and wrapping by arranging the wire 1 in an axial direction of the master B. Both of the methods of wrapping the outer surface 2 of the master B with the wire 1 could allow stent A to be formed in a same manner.

When the wire 1 is wrapped to the outer surface 2 of the master B, plural vertical wires 3 are arranged in an axial direction of the master B and are connected to the wire 1 so as to restrain the position of the wire 1 for the master B in an axial direction. Accordingly, the position in the axial direction and the entire bending shape for the wire 1 being wrapped around the master B are defined by the vertical wires 3. Same as the wire 1, a shape-memorizing alloy of Ni-Ti are used for the vertical wires 3; one through five vertical wires 3 (in this embodiment, three wires) are arranged depending on the conditions of the affected portion C.

When the stent A is bent three dimensionally, it is desirable for positioning the vertical wires 3 at the outer side of the bent shape (the side with large bending radius); more particularly, when the affected portion C is an aneurysm, it is desirable for positioning the vertical wires 3 at the side where the aneurysm is created.

When wrapping the outer surface 2 of the master B with the wire 1, it is necessary for the wire 1 to be stuck closely to the outer surface 2. However, owing to the zigzag shape of the wire 1, sticking closely would be difficult just by wrapping around the outer surface 2. Therefore, after the wire 1 is wrapped around the outer surface 2 of the master B, the wire 1 is stuck closely to the outer surface 2 by tightening a belt-wire 4 made from pure titanium wire over the wire 1.

Subsequently, a shape-memorization process is performed by maintaining the state of having the wire 1 wrapped around the outer surface 2 of the master B, and then by heating. The memorization process is a heating process in which, the wire is maintained for a prescribed time within a vacuum-heating furnace set with a temperature ranging from 400°C through 550°C, and then, the wire is cooled.

In this embodiment the memorization process is performed by combining the following conditions in which, a vacuum degree of the vacuum-heating furnace is set to approximately 10⁻² through 10⁻³ Pa, the temperature is set to 400°C, 450°C, 500°C, 550°C, and the processing time is performed in 30minutes, 60 minutes.

After the memorization process, the wire 1 is detached from the outer surface 2 of the master B to form a stent A capable of returning to the memorized shape under prescribed conditions.

The thus structured stent A is folded in a peripheral direction and diametrally shrunken for being inserted into the delivery kit, and then, is guided into the affected portion C via the blood vessel, and then, returns to the memorized shape reacting to the temperature of body heat when taken out from the delivery kit at the affected portion C. In such occasion, even when the affected portion C is bent or has a diameter transformed in-between, the stent A could stick closely to the inner surface in an extremely steady manner since the stent A is formed already having such conditions taken into consideration.

The outer shape 2 of the master B is formed with a three dimensional shape substantially similar to that of the inner surface of tubular tissue regarding the affected portion C. However, the outer surface 2 is not necessarily required to be substantially similar to the inner surface of the affected portion C, and instead, the inner surface of the master B could be formed substantially similar. In other words, either the outer surface 2 or the inner surface of the master B could be formed with a substantially similar shape to that of the inner surface of the affected portion C.

Further, a metal material, which is capable of enduring to the temperature during the heating of the memorizing process for the wire 1, is required to be used for the master B. As for such material, there are ferrous metal such as stainless steel or carbon steel, or non-ferrous metal such as copper or copper alloy. When manufacturing the master B, it is desirable to use an NC machine tool controlled in accordance with the three dimensional shape-information of the affected portion C.

Since the stent A is of a tubular shape, the master B could also be formed into a tubular shape. That is, a material such as a stainless steel pipe could be used for forming the master B, in which the stainless steel pipe could be processed by bending with a numeric control bending machine or a spinning machine and applied with a drawing process according to circumstance.

Further, when making the master B, machining a metal material is not necessarily required, and instead, a mold of a synthetic resin could be made based on the shape-information of the affected portion C, and the so-called casting method where metal is poured into the mold could be used.

In consequence, in respect of the foregoing stent for this invention, since three dimensional shape-information of a portion targeted for treatment regarding an individual requiring treatment is obtained beforehand by one of the aforementioned methods so as to allow the forming of the stent based on the shape-information, the stent could be stuck closely to the inner wall of the targeted portion in a desirable manner without obstructing the flow of bodily fluid when the stent is positioned in place at the targeted portion for treatment. Further, the stent would not apply excessive force upon the inner wall of the portion targeted for treatment and would not cause necrosis of the inner wall of thus portion.

Further, in respect of another stent regarding this invention, since a memorization process is performed by restraining the shape of the stent corresponding to the master being made in accordance with three dimensional information of the portion targeted for treatment, the stent could return to the memorized shape and back to the original diameter when taken out from the delivery kit after being delivered to the portion targeted for treatment. Subsequently, the stent having returned to the original diameter could stick closely to the inner wall of the portion targeted for treatment in a desirable manner for enabling treatment of thus portion.

Further, in respect of another stent regarding this invention, since the inner surface or the outer surface of the master is formed with a shape substantially similar to the three dimensional shape of the portion targeted for treatment, a stent having memorized the shape of the portion targeted for treatment could be manufactured by performing shape-memorization by restraining the form of the stent in accordance with the master.

The foregoing description of a preferred embodiment of the invention has been presented for purposes of illustration and description, and is not intended to be exhaustive or to limit the invention to the precise form disclosed. The description was selected to best explain the principles of the invention and their practical application to enable others skilled in the art to best utilize the invention in various embodiments and various modifications as are suited to the particular use contemplated. It is intended that the scope of the invention not be limited by the specification, but be defined by the claims set forth below.

## Claims

1. A method of manufacturing a stent (A) so as to manufacture a stent matching with an inner shape of a tubular living-body tissue of each individual by comprising the steps of:
obtaining the inner shape-information regarding the tubular living-body tissue (C) of each individual by using a non surgical method such as roentgen rays, ultrasonic tomogram, computer tomographic image or magnetic resonance image;
making a master (B) in accordance with the shape-information in which the master has a three dimensional inner surface or outer surface substantially similar to the inner shape of the tubular living-body tissue;
shaping the stent (A) in a manner corresponding to the master (B) which bends and bears changes in diameter depending on the individual; and
performing shape-memorization to the stent (A) in a manner corresponding to the master (B)

## Patentansprüche

1. Verfahren zur Herstellung eines Stents, so dass ein Stent hergestellt wird, der an eine innere Form eines schlauch- oder röhrenförmigen Körpergewebes eines jeden Individuums angepasst ist, umfassend die Schritte:
- Erhalten der Informationen über die innere Form des schlauch- oder röhrenförmigen Körpergewebes (C) eines jeden Individuums durch Verwenden eines nicht operativen Verfahrens, wie Röntgenographie, Ultrasonograpie, Computertomographie oder Magnetresonanztomographie;
- Erstellen eines Musters (B) gemäß den Informationen über die innere Form, wobei das Muster eine dreidimensionale innere oder äußere Oberfläche aufweist, die im Wesentlichen der inneren Form des schlauch- oder röhrenförmigen Körpergewebes entspricht;
- Formen des Stents (A) in einer Weise, die dem Model (B) entspricht, welches in Abhängigkeit des Individuums gebogen ist und Änderungen im Durchmesser aufweist; und
- Durchführen einer Formgedächtnisbehandlung des Stents (A) in einer dem Muster (B) entsprechenden Form.

## Revendications

1. Procédé de fabrication d'un stent (A) pour réaliser un stent adapté à la forme intérieure d'un tissu tubulaire de corps humain de chaque individu, comprenant les étapes suivantes :
- obtention d'une information de forme intérieure concernant le tissu tubulaire de corps vivant (C) de chaque individu en utilisant un procédé non chirurgical tel que les rayons X, une tomographie par ultrasons, une image tomographique par ordinateur ou une image par résonance magnétique,
- fabrication d'un modèle (B) selon l'information de forme, le modèle ayant une surface intérieure tridimensionnelle ou une surface extérieure pratiquement analogue à la forme intérieure du tissu tubulaire du corps vivant,
- mise en forme du stent (A) de façon à correspondre au modèle (B) qui se cintre et subit des variations de diamètre suivant l'individu, et
- mémorisation de la forme du stent (A) de façon à correspondre au modèle (B).
